# EUROPEAN PATENT APPLICATION

(11) **EP 3 936 615 A1**
(43) Date of publication of application: **12.01.2022**
(21) Application number: 20765616.6
(22) Date of filing: 04.03.2020
(51) Int. Cl.: C12N 15/11, C12Q 1/04, C12Q 1/68, C12Q 1/6888, G01N 33/50

(54) **METHOD FOR DETERMINING WHETHER ORGANISM HAVING CELL WALL EXISTS AND METHOD FOR IDENTIFYING ORGANISM HAVING CELL WALL**

(30) Priority: 04.03.2019 JP 2019038910
(71) Applicant: Mitsui Chemicals, Inc., Tokyo 105-7122 (JP)
(72) Inventor: FUJII, Ryota, Mobara-shi, Chiba 297-0017 (JP)
(74) Representative: Mewburn Ellis LLP
(86) International application number: PCT/JP2020/009198
(87) International publication number: WO 2020/179823

(57) **Abstract**

A method of determining presence state of an organism having cell wall includes: preparing a liquid sample by immersing a measurement sample in water solvent, or preparing a measurement sample that is water solvent as a liquid sample; and detecting presence state of one or more sRNA species in the liquid sample, wherein presence state of an organism having cell wall is determined based on the presence state of the one or more sRNA species. A method of identifying an organism having cell wall includes: preparing a liquid sample by immersing a measurement sample in water solvent, or preparing a measurement sample that is water solvent as a liquid sample; and detecting presence state of one or more sRNA species in the liquid sample, wherein an organism having cell wall present in the measurement sample is identified based on the presence state of the one or more sRNA species.

SEQUENCE LISTING

<110>  Mitsui Chemicals, Inc.

<120>  METHOD OF DETERMINING EXISTING STATE OF ORGANISM, AND METHOD OF
       IDENTIFYING ORGANISM

<130>  MT-F03299-00

<150>  JP2019-038910
<151>  2019-03-04

<160>  15

<170>  PatentIn version 3.5

<210>  1
<211>  23
<212>  RNA
<213>  Escherichia coli

<400>  1
ugugggcacu cgaagauacg gau                                              23


<210>  2
<211>  19
<212>  RNA
<213>  Escherichia coli

<400>  2
guugugaggu uaagcgacu                                                   19


<210>  3
<211>  20
<212>  RNA
<213>  Escherichia coli

<400>  3
uaacguuaag uugacucggg                                                  20


<210>  4
<211>  20
<212>  RNA
<213>  Pinus thunbergiana

<400>  4
cagaagauag agagcacauc                                                  20


<210>  5
<211>  25
<212>  RNA
<213>  Saccharomyces cerevisiae

<400>  5
gagaucuugg ugguaguagc aaaua                                            25


<210>  6
<211>  70

<212>  DNA
<213>  Artificial Sequence

<220>
<223>  Primer Sequence

<400>  6
ccccaaaaaa tccgtatctt cgagtgccca caaaaaagaa gctgttgtat tgttgtcgaa      60

gaagaaaagt                                                              70


<210>  7
<211>  62
<212>  DNA
<213>  Artificial Sequence

<220>
<223>  Primer Sequence

<400>  7
cccaacccta cccaccctca agaaaaaaaa gtgataattg ttgtcgaaga agaaaaaaaa      60

tt                                                                      62


<210>  8
<211>  18
<212>  DNA
<213>  Artificial Sequence

<220>
<223>  Primer Sequence

<400>  8
gaagctgttg ttatcact                                                     18


<210>  9
<211>  71
<212>  DNA
<213>  Artificial Sequence

<220>
<223>  Primer Sequence

<400>  9
ccccaaaaag gagcgatgtg ctctctatct tctgaaaaga agctgttgta ttgttgtcga      60

agaagaaaag t                                                            71


<210>  10
<211>  19
<212>  RNA
<213>  Fusarium oxysporum

<400>  10
uccgguaugg uguaguggc                                                    19


<210>  11
<211>  17

<212>  RNA
<213>  Escherichia coli

<400>  11
uuugcucuuu aaaaauc                                                    17


<210>  12
<211>  22
<212>  RNA
<213>  Escherichia coli

<400>  12
cucgaagaua cggauucuua ac                                              22


<210>  13
<211>  17
<212>  DNA
<213>  Artificial Sequence

<220>
<223>  Primer Sequence

<400>  13
ccgggaacgt attcacc                                                    17


<210>  14
<211>  20
<212>  DNA
<213>  Artificial Sequence

<220>
<223>  Primer Sequence

<400>  14
agagtttgat catggctcag                                                 20


<210>  15
<211>  17
<212>  DNA
<213>  Artificial Sequence

<220>
<223>  Primer Sequence

<400>  15
ccgggaacgt attcacc                                                    17

## Description

### Technical Field

The present disclosure relates to a method of determining the presence state of an organism having a cell wall, and a method of identifying an organism having a cell wall.

### Background Art

Control of organisms, such as bacteria, that have undesirable effects on the human body is important in places requiring hygiene management, such as food production, medical care, welfare, and household. Most of bacteria in the environments are harmless, but some bacteria cause food poisoning, product spoilage or deterioration, and can be a major hindrance in people's lives.

Recently, attempts have been more widely made which includes detecting bacteria by certain methods (so-called "visualization") and use the detection results as indices of hygiene management concerning bacteria. For bacteria detection, the most basic detection methods are culture methods. A culture method is a method including culturing bacteria on a medium, and detecting the bacteria based on the morphology of the bacteria.

Methods for detecting microorganisms other than culture methods include methods in which surface antigens of the microorganisms are recognized by antibodies (antibody methods). Representative examples of antibody methods include the immunochromatography method, the latex agglutination method, and the ELISA method.

Methods for detecting microorganisms other than the above-described methods include methods in which microorganisms are detected based on the genes contained in the microorganisms (genetic methods). A genetic method is a method in which the presence or absence of a nucleic acid sequence of a part of a gene contained in the microorganism to be detected is examined to identify the microorganism present. Examples of a nucleic acid molecule containing a nucleic acid sequence used for identification include DNA, and ribosome-derived RNA called 16S rRNA. For example, in Japanese Patent Application Laid-Open (JP-A) No. 2013-93, *Mycobacterium avium* and *Mycobacterium intracellulare* are detected based on the nucleotide sequence of 16S rRNA.

DNA is a nucleic acid that almost all organisms possess, and 16S rRNA is a nucleic acid that almost all organisms possess. These nucleic acids are present in large amounts in living organisms. Thus, DNA is a suitable target for detection and has been conventionally used in studies regarding detection of microorganisms based on genetic sequences. Also, 16S rRNA is a suitable target for detection and has been conventionally used in studies regarding detection of microorganisms based on genetic sequences. DNA sequences include a part of which sequence is conserved among organisms and a part of which sequence varies among organisms. An organism can be identified based on a part of which sequence varies among organisms. 16s rRNA sequences include a part of which sequence is conserved among organisms and a part of which sequence varies among organisms. An organism can be identified based on a part of which sequence varies among organisms.

### SUMMARY OF THE INVENTION

### Problem to be solved by invention

An embodiment of the present disclosure aims to provide a determination method that enables simple determination of the presence state of an organism in a measurement sample, and an identification method that enables simple identification of an organism contained in a measurement sample.

### Means for solving the problem

<1> A method of determining the presence state of an organism having a cell wall, the method including:
   preparing a liquid sample by immersing a measurement sample in a water solvent, or preparing a measurement sample that is a water solvent as a liquid sample; and
   detecting the presence state of one or more sRNA species in the liquid sample,
   wherein the presence state of an organism having a cell wall is determined based on the presence state of the one or more sRNA species.
<2> The determination method according to < 1 >, wherein the one or more sRNA species exhibit a specific expression profile in the organism having a cell wall.
<3> The determination method according to <1> or <2>, wherein determining the presence state of an organism having a cell wall includes determining a gross presence state of two or more organisms having a cell wall.
<4> The determination method according to any one of <1> to <3>, wherein detecting the presence state of the sRNA species includes detecting the abundance of the sRNA species.
<5> The determination method according to any one of <1> to <4>, wherein the organism having a cell wall includes at least one selected from the group consisting of *Escherichia coli, Citrobacter ƒreundii*, and *Salmonella gallinarum.*
<6> The determination method according to any one of <1> to <4>, wherein the organism having a cell wall includes a plant.
<7> The determination method according to any one of <1> to <4>, wherein the organism having a cell wall includes a verotoxin-producing bacterium.
<8> The determination method according to any one of <1> to <7>, wherein the measurement sample is obtained by collecting airborne matter, and determining the presence state of an organism having a cell wall includes determining the presence state of an organism having a cell wall present in the air.
<9> The determination method according to any one of <1> to <8>, wherein immersing the measurement sample is performed at a temperature in a temperature range of from 0°C to 50°C.
<10> The determination method according to any one of <1> to <9>, wherein each of the one or more sRNA species has a length in a range of from 5 to 500 bases.
<11> The determination method according to any one of <1> to <10>, wherein the one or more sRNA species include at least one selected from the group consisting of EC-5p-36 having the nucleotide sequence of SEQ ID NO: 1, EC-3p-40 having the nucleotide sequence of SEQ ID NO: 2, EC-5p-79 having the nucleotide sequence of SEQ ID NO: 11, EC-3p-393 having the nucleotide sequence of SEQ ID NO: 12, fox_milRNA_5 having the nucleotide sequence of SEQ ID NO: 10, miR156 having the nucleotide sequence of SEQ ID NO: 4, and miR716b having the nucleotide sequence of SEQ ID NO: 5.
<12> The determination method according to any one of <1> to <11>, wherein the water solvent contains a nucleic acid amplifying reagent.
<13> The determination method according to any one of <1> to <12>, wherein detecting the presence state of the one or more sRNA species is performed using isothermal gene amplification.
<14> The determination method according to <13>, wherein the isothermal gene amplification is performed at a temperature in a temperature range of from 10°C to 40°C.
<15> The determination method according to any one of <1> to <12>, wherein detecting the presence state of the one or more sRNA species is performed using a PCR.
<16> A method of identifying an organism having a cell wall, the method including:
   preparing a liquid sample by immersing a measurement sample in a water solvent, or preparing a measurement sample that is a water solvent as a liquid sample; and
   detecting the presence state of one or more sRNA species in the liquid sample,
   wherein an organism having a cell wall present in the measurement sample is identified based on the presence state of the one or more sRNA species.
<17> The identification method according to <16>, wherein the one or more sRNA species exhibit a specific expression profile in accordance with an organism having a cell wall.
<18> The identification method according to <16> or <17>, wherein identifying an organism having a cell wall present in the measurement sample includes identifying that at least one of two or more candidate organisms is present.
<19> The identification method according to any one of <16> to < 18>, wherein detecting the presence state of the sRNA species includes detecting the abundance of the sRNA species.
<20> The identification method according to any one of <16> to <19>, wherein the measurement sample is obtained by collecting airborne matter, and wherein identifying an organism having a cell wall includes identifying an organism having a cell wall present in the air.
<21> The identification method according to any one of <16> to <20>, wherein immersing the measurement sample is performed at a temperature in a temperature range of from 0°C to 50°C.
<22> The identification method according to any one of <16> to <21>, wherein each of the one or more sRNA species has a length in a range of from 5 to 500 bases.
<23> The identification method according to any one of <16> to <22>, wherein the one or more sRNA species include at least one selected from the group consisting of EC-5p-36 having the nucleotide sequence of SEQ ID NO: 1, EC-3p-40 having the nucleotide sequence of SEQ ID NO: 2, EC-5p-79 having the nucleotide sequence of SEQ ID NO: 11, EC-3p-393 having the nucleotide sequence of SEQ ID NO: 12, fox_milRNA_5 having the nucleotide sequence of SEQ ID NO: 10, miR156 having the nucleotide sequence of SEQ ID NO: 4, and miR716b having the nucleotide sequence of SEQ ID NO: 5.
<24> The identification method according to any one of <16> to <23>, wherein the water solvent contains a nucleic acid amplifying reagent.
<25> The identification method according to any one of <16> to <24>, wherein detecting the presence state of one or more sRNA species is performed using isothermal gene amplification.
<26> The identification method according to <25>, wherein the isothermal gene amplification is performed at a temperature in a temperature range of from 10°C to 40°C.
<27> The identification method according to any one of <16> to <24>, wherein detecting the presence state of one or more sRNA species is performed using a PCR.

### Effect of invention

According to the present disclosure, a determination method that enables simple determination of the presence state of an organism having a cell wall in a measurement sample, and an identification method that enables simple identification of an organism having a cell wall contained in a measurement sample are provided.

### BRIEF DESCRIPTION OF THE DRAWINGS

Fig. 1 shows the results of detection of *E. coli*-derived sRNA by isothermal gene amplification in Example 4.
Fig. 2 shows the results of detection of black pine pollen-derived sRNA by isothermal gene amplification in Example 5.
Fig. 3 shows the results of electrophoresis of nucleic acid liquid extract in Example 10.

### DESCRIPTION OF EMBODIMENTS

The present disclosure will be described in detail below. In the following, the explanation of constituent elements may be made based on representative embodiments of the present disclosure. However, the present disclosure is not limited to such embodiments.

In a series of numerical ranges described in the present disclosure, the upper or lower limit value of one numerical range may be replaced by the upper or lower limit value of another numerical range in the series of numerical ranges. Further, the upper or lower limit value of a numerical range described in the present disclosure may be replaced by a value described in the working examples.

In a case in which plural substances corresponding to a component of interest are contained, the content of the component described in the present disclosure means the total amount of the plural substances contained, unless otherwise specified.

In the present disclosure, the terms " mass%" and " weight%" are used synonymously, and the terms "parts by mass" and "parts by weight" are used synonymously.

In the present disclosure, two or more exemplary aspects that are separately described may be combined with each other to configure a new aspect as long as the aspects do not contradict each other.

Among methods for detecting organisms, culture methods are very sensitive and highly reliable methods. However, culture methods involve some problems. Examples of problems associated with culture methods include: the considerable time that it takes to obtain results due to the necessity of cultivation for one to several days; the difficulty in application to bacteria of which cultivation is difficult; the necessity of special techniques for identification of bacteria after cultivation; and the burden and costs of disposal due to the necessity for sterilization treatment, such as autoclaving, for disposal of cultured bacteria and the bulkiness of the volume of the waste.

In the case of antibody methods, for example, the immunochromatography method and the latex agglutination method have the advantage of simplicity of procedures, since microorganisms can be detected simply by contacting a sample. However, the immunochromatography method and the latex agglutination method have problems in terms of low detection sensitivity, which allows detection only when microorganism cells are present at high concentrations. The ELISA method has higher sensitivity than that of the immunochromatography method or the like. However, the ELISA method is burdensome due to the necessity of washing and color development.

Conventional genetic methods lack simplicity since procedures to extract DNA or 16s rRNA from a microorganism by denaturing the cell membrane are burdensome. Specifically, since DNA or 16s rRNA is confined in cells by cell membranes of microorganisms, and it is considered difficult to measure it directly. In order to analyze DNA and 16S rRNA, procedures are required which include denaturing cell membranes with a strong denaturing agent (a denaturing agent that is strong enough to denature cell membranes) such as phenol, a guanidine salt, or sodium hydroxide for nucleic acid extraction, and then removing or neutralizing the denaturing agent. Thus, in conventional genetic methods, treatment with a denaturing agent and removal or neutralization of the denaturing agent are burdensome, as a result of which the methods are more complicated and less convenient than culture methods and the immunochromatography method. There is also a method in which nucleic acids are extracted from cells by heating. One example of this method includes heating a bacteria-containing solution at 100°C for 10 minutes, and using the resultant for nucleic acid amplification. However, the heat treatment requires special equipment and also raises the necessity to pay attention to safety during heating.

In consideration of the above situation, the present inventors have carried out intensive study, as a result of which the present inventors have found that sRNAs in the cell can be extracted by immersing a cell of an organism having a cell wall in water, or in an aqueous solution that contains water and additional components in a range in which a cell denaturation effect is not significantly elevated. This finding is surprising for the following reason. Regarding a cell of an organism that does not have a cell wall, it can be possible to extract DNA and 16s RNA to the surrounding environment by immersing the cell in a hypotonic solution, through rupture of the cell membrane due to osmotic pressure, even without using the denaturing agent described above. However, it would be considered, based on conventional common technical knowledge, this method cannot be applied to a cell of an organism having a cell wall. More specifically, in a cell of an organism having a cell wall, the cell wall thereof maintains the structure of the cell; therefore, the cell membrane of the cell is not ruptured by osmotic pressure changes in the surrounding environment, and the cell is more resistant to lysis. Considering the foregoing, it would be considered, based on conventional common technical knowledge, that it is difficult to cause nucleic acids present in a cell of an organism having a cell wall to be released into the surrounding environment unless the above-described procedures to denature the cell membrane are taken. Therefore, it is surprising that sRNAs can be extracted from a cell of an organism having a cell wall to the outside of the cell by immersing the cell in water.

A method of determining the presence state of an organism having a cell wall according to the present disclosure includes:
preparing a liquid sample by immersing a measurement sample in a water solvent, or preparing a measurement sample that is a water solvent as a liquid sample; and
detecting the presence state of one or more sRNA species in the liquid sample,
wherein the presence state of an organism having a cell wall is determined based on the presence state of the one or more sRNA species (hereinafter also referred to as simply "determination method according to the present disclosure"). The method of identifying an organism having a cell wall according to the present disclosure includes:
   preparing a liquid sample by immersing a measurement sample in a water solvent, or preparing a measurement sample that is a water solvent as a liquid sample; and
   detecting the presence state of one or more sRNA species in the liquid sample,
   wherein an organism having a cell wall present in the measurement sample is identified based on the presence state of the one or more sRNA species (hereinafter also referred to as simply "identification method according to the present disclosure").

The determination method according to the present disclosure enables a simple determination of the presence state of an organism having a cell wall to be detected in a measurement sample. The identification method according to the present disclosure enables a simple identification of the organism having a cell wall present in a measurement sample. In the determination method according to the present disclosure and the identification method according to the present disclosure, there is no necessity to use a strong denaturing agent such as phenol, a guanidine salt, an ionic surfactant, an alcohol, or sodium hydroxide, and the determination or identification can be carried out in a simple manner.

The determination method according to the present disclosure and the identification method according to the present disclosure can be collectively expressed as the following method; specifically, a method including:
preparing a liquid sample by immersing a measurement sample in a water solvent, or preparing a measurement sample that is a water solvent as a liquid sample; and
detecting the presence state of one or more sRNA species in the liquid sample,
wherein the presence state of an organism having a cell wall in the measurement sample or identification of an organism present in the measurement sample is obtained based on the presence state of the one or more sRNA species (hereinafter also referred to as "Method A according to the present disclosure").

The more specific embodiments described in the "Means for solving the problem" section can also be applied to Method A described above.

Organisms contain short RNA fragments called small RNAs. Small RNAs (hereafter also referred to as "sRNAs") play an important role in the regulation of biological processes such as development, differentiation, transposon silencing, and viral protection. Since sRNAs have important functions in a cell, sRNAs expressed exhibit nucleotide sequence conservation within the same organism, i.e., plural cells of the same organism has a common expression profile with respect to various sRNAs. However, when different organisms are compared, the expression profile of sRNAs is a differential expression profile in accordance with the organism species. Therefore, the information of sRNAs present in a measurement sample can be used to identify the organism, as with DNA and 16S rRNA. It has been found, for the first time, by the present disclosure that identification of an organism can be carried out using sRNAs.

For example, assuming that sRNA (A) having a nucleotide sequence (A) is expressed in organism (A) whereas sRNA (B) having a nucleotide sequence (B) is not expressed in organism (A), and that sRNA (B) is expressed in organism (B) whereas sRNA (A) is not expressed in organism (B), the determination that cells of organism (A) are present but cells of organism (B) are not present can be made if the presence of sRNA (A) is detected and the presence of sRNA (B) is not detected in the measurement sample. However, assuming that sRNA (A) is also expressed in organism (C) that is other than organisms (A) and (B), determination that cells of at least one of organism (A) or organism (C) are present but cells of organism (B) are not present can be made if the presence of sRNA (A) is detected but the presence of sRNA (B) is not detected in a measurement sample.

Although the existence of sRNAs has already been known, it has been thought that, as with DNA and 16S rRNA described above, sRNA cannot be taken out of a cell having a cell wall unless a strong denaturing agent is used. However, in the present disclosure, it is surprisingly found that when a cell having a cell wall is placed in a water solvent, sRNAs in the cell are extracted in the water solvent around the cell (i.e., escapes into the water solvent) even when the cell membrane is not destroyed with, for example, a strong denaturing agent. This extraction can be carried out even at room temperature. The sRNAs extracted in the water solvent can be used for detecting the presence of a specific organism of interest or for identifying an organism present in the measurement sample. Therefore, according to the present disclosure, a specific organism of interest can be detected and the identity of an organism present in a measurement sample can be identified in a simple manner without burden.

Since sRNAs have a small strand length, sRNAs are more resistant to attack by RNase than 16S rRNAs, and enable more stable analysis. In addition, since the copy number of a sRNA in a cell is as large as several thousands to several tens of thousands in the case of a sRNA having a larger copy number, it is possible to obtain information on the presence state of a sRNA can be obtained with high sensitivity.

In the following, the determination method according to the present disclosure and the identification method according to the present disclosure are described together. The descriptions below shall apply to both of the determination method according to the present disclosure and the identification method according to the present disclosure, and shall apply also to Method A according to the present disclosure.

### <Organism Having Cell Wall>

In the determination method according to the present disclosure and the identification method according to the present disclosure, the organism having a cell wall is not particularly restricted, and may be any organism having a cell wall. In general, cells other than animal cells, such as plant cells and microbial cells, have a cell wall. The organism having a cell wall may be a fungus such as yeast, slime mold, or mold, or may be a bacterium such as *E*. *coli.* The object to be determined or identified is not necessarily the whole organism body, and may be a part of an organism body. This is because sRNAs are also present in a part of an organism body, and the detection of a part of an organism body also provides information on the presence state of the organism. The part of an organism body described above may be a part of a multicellular organism body, for example, pollen of a plant. The part of an organism body is preferably a part in which cell shapes are retained. This is because in a case in which cellular shapes are not retained in the part of an organism body, it is possible that intracellular components have already escaped into the surrounding medium prior to immersion in a water solvent or prior to preparation of a measurement sample that is a water solvent. As described above, it is described, in the determination method according to the present disclosure, that the presence state of an organism having a cell wall is determined, it is described, in the identification method according to the present disclosure, that an organism having a cell wall is identified; the scope of the organism having a cell wall in these descriptions shall encompass a part of an organism body. Thus, the determination of the presence state or the identification is only required to provide information regarding an organism or a group of organisms. In fact, it is considered that a material contained in a measurement sample in the case of detection of, for example, a herbaceous plant is generally a part of an organism body rather than the whole organism body. Nevertheless, it is possible to obtain a determination or identification result regarding the presence of an organism or a group of organisms by using the determination method according to the present disclosure or the identification method according to the present disclosure. In view of the above, the organism in the determination method according to the present disclosure and the identification method according to the present disclosure may be a plant, in which case a component contained in the measurement sample may be, for example, pollen.

The organism having a cell wall may be, for example, an organism of which presence may cause a problem in terms of hygiene management at sites at which hygiene management is necessary (for example, food production, medical care, welfare, and household). Such organisms include pathogenic microorganisms and putrefactive microorganisms. Pathogenic microorganisms may be pathogenic fungi, examples of which include *Trichophyton*, *Candida*, and *Aspergillus.* Pathogenic microorganisms may be pathogenic bacteria, examples of which include Gram-positive bacteria (for example, *Staphylococcus*, *Streptococcus*, *Streptococcus pneumoniae, Enterococcus, Diphtheria, Mycobacterium tuberculosis, Mycobacterium leprae, Bacillus anthracis, Bacillus subtilis, Clostridium perfringens, Clostridium tetani*, and *Clostridium botulinum*), and Gram-negative bacteria (for example, *Neisseria gonorrhoeae, Neisseria meningitidis, Salmonella enterica, Escherichia coli , Pseudomonas aeruginosa, Shigella, Haemophilus influenzae, Bordetella pertussis, Vibrio cholerae*, *Vibrio parahaemolyticus* , *Acinetobacter*, *Campylobacter*, *Legionella*, and *Helicobacter*). Pathogenic bacteria may be verotoxin-producing bacteria. Examples of putrefactive microorganisms include bacteria of various genera, such as *Pseudomonas*, *Micrococcus*, *Vibrio*, and *Flavobacterium* bacteria in the case of seafoods, *Pseudomonas*, *Achromobacter*, *Micrococcus*, and *Flavobacterium* bacteria in the case of meats, and *Bacillus* bacteria in the case of rice and noodles.

In an embodiment, the organism having a cell wall preferably includes at least one selected from the group consisting of *Escherichia coli, Citrobacter freundii,* and *Salmonella gallinarum.* In another embodiment, the organism having a cell wall includes a plant, and the plant may be black pine. In still another embodiment, the organism having a cell wall includes a verotoxin-producing bacterium.

Since sRNAs that have escaped from a cell having a cell wall into a water solvent is used for detection in the determination method according to the present disclosure and the identification method according to the present disclosure, a treatment for removing a substance that impedes such escape in advance may be performed.

### <Measurement Sample>

The measurement sample in the determination method according to the present disclosure is a sample that is used for the determination of the presence state of an organism having a cell wall to be detected. The measurement sample in the identification method according to the present disclosure is a sample that is used for the identification of an organism having a cell wall. The measurement sample is not particularly limited as long as the measurement sample prepared contains an organism having a cell wall in a case in which an object regarding which analysis of the presence state of an organism having a cell wall is desired (hereinafter also referred to as "analysis object") contains the organism having a cell wall. The measurement sample may be the analysis object itself, or a sample prepared from the analysis object by any process. The analysis object is, for example, a surface of an object such as a surface of a workbench, an object itself such as food, a liquid such as tap water, a gas such as air, or a bacterium of which identity has not been identified. The measurement sample is preferably an analysis object itself from the viewpoint of simplifying the procedures.

For example, when analysis of an organism having a cell wall present in a liquid as an analysis object is desired, the measurement sample may be the liquid itself, or a sample obtained by subjecting the liquid to a treatment such as dilution. For example, when analysis of an organism present on a surface of an object (for example, a surface of a workbench) as an analysis object is desired, the surface may be wiped with a wipe, cotton swab, or the like, and then the wipe, the cotton swab, or the like, or a part thereof, may be used as the measurement sample.

When analysis of an organism having a cell wall present within an object (for example, within a food) as an analysis object is desired, the entire object or a part of the object may be used as the measurement sample, or the object may be immersed in a liquid and the obtained liquid may be used as the measurement sample. When the air object is the air and analysis of an organism present in the air is desired, a sample obtained by collecting airborne matter present in the air may be used as the measurement sample. Collecting the airborne matter can be carried out by, for example, filtering, centrifugation (for example, cyclonic separation), or simply allowing an open container to stand still (for example, by allowing a Petri dish or the like to stand still while maintaining its lid open).

As described above, the measurement sample can be, for example: a liquid as an analysis object; a sample obtained by diluting the liquid; a wipe, a cotton swab, or the like with which a surface as an analysis object has been wiped; collected matter collected on a filter used for filtering the air; or attached matter attaching to a container that has been left open to the ambient atmosphere. Alternatively, for the purpose of, for example, identifying the organism having a cell wall that has already been obtained, it is also possible to use the organism itself as the measurement sample.

### <Water Solvent>

The water solvent in the determination method according to the present disclosure and the identification method according to the present disclosure is a liquid of which the main component is water. The water solvent may be pure water itself, or an aqueous solution containing other components in addition to water (hereinafter also referred to as "coexisting components") as long as the other components do not cause denaturation of the cell membrane. The amount of solvent components other than water in the water solvent is preferably as small as possible. The amount of solvent components other than water may be 1 mass% or less, 0.1 mass% or less, 0.01 mass% or less, 0.001 mass% or less, or 0 mass% by mass (i.e., the solvent components include only water) with respect to the amount of water. The "water solvent" in the present disclosure does not contain any components that denature and destroy the cell membrane, or the water solvent contains components that denature and destroy the cell membrane only in an amount that is so small as not to cause denaturation of the cell membrane. In other words, the water solvent has a basic property in common with water in that the water solvent does not cause denaturation of the cell membrane, and the water solvent may contain coexisting components as long as this basic property is not impaired. Thus, the water solvent according to the present disclosure does not contain extractants for destroying cell membranes and extracting intracellular components (for example, EXTRAGEN (Tosoh Corporation), a cell component extractant referred to in JP-A No. 2013-93).

The pH of the water solvent is preferably in the range from pH 5 to 9, more preferably from pH 6 to 8, and even more preferably from pH 6.5 to 7.5, from the viewpoint of preventing denaturation of the cell membrane occurring in a strongly acidic or strongly alkaline condition. The water solvent may contain coexisting components other than water, as described above. The total amount of the coexisting components may be 30 mass% or less, 10 mass% or less, 1 mass% or less, 0.1 mass% or less, 0.01 mass% or less, or 0.001 mass% or less with respect to the total amount of the water solvent. Examples of coexisting components that may be contained in the water solvent include salts, buffers, surfactants, DTT, and RNase inhibitors. Although sRNAs, having a small strand length, are less likely to be attacked by RNase than longer RNAs such as 16S rRNA, sRNAs can be more stabilized by allowing RNA stabilizers, such as DTT and RNase inhibitors, to coexist with the sRNAs.

The water solvent does not contain coexisting components in such an amount as to denature and destroy the cell membrane. Preferably, the water solvent does not contain phenol, guanidine, alcohols, ionic surfactants, or strong alkalis such as NaOH, from the viewpoint of preventing denaturation of the cell membrane. When the water solvent contains denaturing components such as phenol, guanidine, alcohols, ionic surfactants, and strong alkalis such as NaOH, the total amount of the denaturing components is preferably 0.1 mass% or less, more preferably 0.01 mass% or less, and still more preferably 0.001 mass% or less, with respect to the total amount of the water solvent. When the water solvent does not contain any denaturing components, such as strong alkalis, or contains denaturing components in an amount within the foregoing range, it is not necessary to perform an additional treatment to remove the denaturing components in order to perform further processing after the preparation of the liquid sample by immersion, as a result of which identification of an organism having a cell wall can be carried out in a simpler manner. The requirement that denaturing components should not be contained or the amount of denaturing components should be within the foregoing range even when contained is also preferred from this viewpoint.

Since surfactants may denature and destroy the cell membrane depending on the type and amount of the surfactants, it is necessary restrict the type and amount of the surfactants when the water solvent contains surfactants. The surface tension of the water solvent at 20°C is preferably from 50 mN/m to 72.8 mN/m (surface tension of water), more preferably from 60 mN/m to 72.8 mN/m, still more preferably from 65 mN/m to 72.8 mN/m, and even more preferably 70 mN/m to 72.8 mN/m. When the water solvent contains a surfactant, the surfactant is preferably a neutral surfactant, and examples of the neutral surfactant include Tween series surfactants (for example, Tween-20), NP-40, and Triton series surfactants (for example, Triton X-100).

The salt concentration in the water solvent is preferably from 0 mol/L to 0.2 mol/L, more preferably from 0 mol/L to 0.1 mol/L, and still more preferably from 0 mol/L to 0.05 mol/L, from the viewpoint of allowing the water solvent to have properties that are similar to those of pure water.

The coexisting component does not need to be a solvent component, and may be, for example, fine particles suspended in water, or a water-soluble substance. The water solvent may also contain a nucleic acid amplifying reagent as a coexisting component. The nucleic acid amplifying reagent is a reagent that is necessary for amplification of nucleic acid and that includes, for example, a polymerase, nucleotide triphosphates (a mixture of dNTPs), primers, and Mg²⁺ ions. Preferably, the nucleic acid amplifying reagent includes a polymerase having an activity capable of synthesizing a DNA strand using an RNA as the template (reverse transcription activity). The nucleic acid amplifying reagent may include a weak surfactant (especially, a neutral surfactant), such as Triton X-100. Since the surfactant contained in the nucleic acid amplifying reagent will not denature cells and destroy the membrane at the concentration used in the nucleic acid amplifying reagent, the water solvent may contain the surfactant as it is. For example, when the water solvent contains Triton X-100, the content of Triton X-100 is preferably from 0.01 mass% to 5 mass%, more preferably from 0.05 mass% to 3 mass%, and still more preferably from 0.1 mass% to 2 mass%, with respect to the total amount of the water solvent, from the viewpoint of preventing denaturation of the cell membrane and efficiently performing nucleic acid amplification. The nucleic acid amplifying reagent may be, for example, a PCR reagent, or an isothermal gene amplification reagent. When the water solvent contains a nucleic acid amplifying reagent, a liquid sample containing sRNAs that have escaped from cells having a cell wall can be used, as it is, for nucleic acid amplification without carrying out a reagent addition operation or a temperature cycling operation.

### <Immersing>

Immersing in the determination method according to the present disclosure and the identification method according to the present disclosure may be performed at room temperature, or may be performed while being heated or cooled. In order to minimize the denaturation of the cell membrane, immersing is preferably performed at a temperature in a temperature range of from 0°C to 50°C, more preferably performed at a temperature in a temperature range of from 4°C to 40°C, still more preferably performed at a temperature in a temperature range of from 10°C to 40°C, even more preferably performed at a temperature in a temperature range of from 20°C to 40°C, further more preferably performed at a temperature in a temperature range of from 25°C to 40°C, and still further more preferably performed at a temperature in a temperature range of from 30°C to 37°C. Immersing may be performed at room temperature.

The immersing time is not particularly limited as long as escape of sRNA to the outside of cells occurs in an amount sufficient for detection. The immersing time is, for example, from 10 seconds to 30 hours, and may be from 10 seconds to 10 hours, from 1 minute to 5 hours, or from 5 minutes to 1 hour. Alternatively, the immersing time may be from 0.5 hours to 6 hours, from 0.7 hours to 4.5 hours, or from 0.8 hours to 2 hours. The manner of immersing is not particularly limited, and examples thereof include any treatment in which an organism having a cell wall, if any, contained in the measurement sample is brought into contact with the water solvent. When the measurement sample is a solid sample (for example, a cotton swab, a wipe, a filter, an analysis object itself or a part of the analysis object, or microbial cells), immersing can be performed, for example, by immersing the solid sample as the measurement sample in a water solvent contained in a container.

Since an organism having a cell wall can itself be used as the measurement sample as described above, an organism having a cell wall in an independent state may be immersed in the water solvent, or an organism having a cell wall attaching to another article may be immersed in the water solvent by immersing the article in the water solvent.

By carrying out the immersing, sRNAs escape to the outside of the cells in a case in which an organism having a cell wall is present in the measurement sample, and a liquid sample containing sRNAs in a water solvent can be obtained.

### <Measurement Sample That Is Water Solvent>

When the analysis object is in the state of a water solvent from the beginning, the analysis object itself can be considered as a measurement sample and can be directly used as a liquid sample. For example, by using tap water, as it is, as a liquid sample, the presence state of an organism having a cell wall in the tap water can be determined, or an organism having a cell wall contained in the tap water can be identified. Since the analysis object potentially contains an organism having a cell wall, the water solvent as a measurement sample described above potentially contains the organism having a cell wall.

When the analysis object is a liquid, but pretreatment of the analysis object is desired for a reason such as the presence of a large amount of contaminants, a measurement sample that is a water solvent may be prepared by a technique of, for example, removing the contaminants by filtration, centrifugation, dialysis, or the like, and the measurement sample prepared may be used as a liquid sample. For example, when an organism having a cell wall in muddy water is to be tested, mud in the muddy water may be removed, for example, by filtration, and then the presence state of the organism having a cell wall can be determined or the organism having a cell wall can be identified by using the filtrate (a measurement sample potentially containing an organism having a cell wall) as a liquid sample. Since it is permissible for the water solvent to contain coexisting components other than water as described above, the organism having a cell wall that is potentially contained in the measurement sample is potentially contained as a coexisting component in the water solvent.

A configuration in which the water solvent does not contain any coexisting components other than organisms having a cell wall described above is also a preferable embodiment. By allowing organisms having a cell wall that are potentially contained in the measurement sample to contact the water solvent for a certain length of time (for example, the length of time illustrated as examples of the immersing time described above), sRNAs escape from the organisms (in a case in which the measurement sample actually contains the organisms having a cell wall), and a liquid sample containing sRNAs, which is similar to that obtained in the case of carrying out the immersing described above, can be obtained. The aspect in which a measurement sample that is a water solvent is used can be used, for example, for determination of the presence state of an organism having a cell wall in water (for example, tap water, or sewage) as an analysis object, or for identification of an organism having a cell wall contained in water (for example, tap water, or sewage).

As far as an organism having a cell wall contacts a water solvent in an operation, the operation is included in the scope of the aforementioned preparation of a liquid sample by immersing a measurement sample in a water solvent or the aforementioned preparation of a measurement sample that is a water solvent as a liquid sample. In a case in which a liquid sample is prepared by immersing a measurement sample in a water solvent, the operation may be an operation of artificially allowing time to elapse for the purpose sRNA extraction while allowing the water solvent in which the measurement sample is immersed to stand still or while carrying out, for example, stirring. Similarly, in a case in which the measurement sample is a liquid sample, the operation may be an operation of artificially allowing time to elapse for the purpose of sRNA extraction while allowing the liquid sample to stand still or while carrying out, for example, stirring.

Surprisingly, the escape of sRNAs from a cell having a cell wall, which is described above in the "Immersing" and "Measurement Sample as Water Solvent" sections, is not a phenomenon that occurs with nucleic acids in general, but is a phenomenon that occurs specially with sRNAs. Therefore, for example, even when cells having a cell wall are immersed in a water solvent with a desire to cause 16S rRNA, which has been conventionally used to identify organisms, to escape into the water solvent, 16S rRNA exhibits no escape from the cells having a cell wall, or, even when 16S rRNA escapes from the cells, the amount that escapes is so low as to be insufficient for the detection of organism species. We consider that one reason for this difference may be a difference in length between 16S rRNA (about 1,600 bases) and sRNAs. That is, sRNAs, which have a smaller strand length than mRNAs and 16S rRNA, surprisingly escape into the water solvent outside the cells having a cell wall even when the cell membrane is not disrupted. In contrast, larger molecules such as mRNAs and 16S rRNA do not substantially exhibit such escape, and cannot be taken out of cells having a cell wall unless an extraction operation that is accompanied by denaturation of the cell membrane is carried out.

### <sRNA>

A sRNA in the determination method according to the present disclosure and the identification method according to the present disclosure is also referred to as a small RNA, and means a short-strand RNA contained in a cell. The specific strand length of the sRNA is preferably from 5 bp to 500 bp, more preferably from 8 bp to 500 bp, still more preferably from 10 bp to 200 bp, even more preferably from 12 bp to 100 bp, and particularly preferably from 15 bp to 30 bp.

The scope of sRNAs includes micro RNAs contained in eukaryotic cells, which are RNAs having about 20 bp to about 30 bp. Also, particularly short RNAs in prokaryotic organisms having strand lengths comparable to microRNAs are sometimes called microRNA-size small RNAs.

Studies have been carried out with respect to sRNAs present in organisms having a cell wall, and the sRNA sequence information found in organisms has been accumulated in databases such as Rfam (EMBL EBI), Small RNA Database (MD Anderson Cancer Center), miRBase (Griffiths-Jones lab at the Faculty of Biology, Medicine and Health, University of Manchester), and National Center for Biotechnology Information (NCBI) database, and has also been reported in various academic papers. Thus, information on sRNA species contained in various organisms can be obtained by known methods such as database search (see Journal of the Kyorin Medical Society, 41(1), pp. 13-18, April 2010). For example, by using Nucleotide BLAST (https://blast.ncbi.nlm.nih.gov/Blast.cgi), a nucleic acid sequence included in the National Center for Biotechnology Information (NCBI) database and a nucleic acid sequence for which search is to be carried out can be compared with each other, and search for an identical sequence (including information on the organism from which the identical sequence comes) can be performed.

Further, sRNA species exhibit differential expressions in accordance with the type of organism. For example, by referring to sequence databases such as Rfam, Small RNA Database, miRBase, and National Center for Biotechnology Information (NCBI) database, it is possible to find out, by searching, one or more organisms in which a particular sRNA species is expressed, and find out, by searching, one or more sRNA species that are expressed in a particular organism. In the determination method according to the present disclosure, the one or more sRNA species preferably exhibit a specific expression profile in a particular organism of interest. With such a specific expression profile, the presence state of the organism can be better determined based on the presence state of the sRNA species.

The term "specific expression profile" as used herein does not necessarily refer to a sRNA species that is expressed only in the specific organism having a cell wall or a sRNA species that shows no expression only in the specific organism having a cell wall. Instead, the term means a concept encompassing a sRNA species other than a sRNA completely specific only to the specific organism having a cell wall, the concept encompassing, for example, a sRNA species expressed only in a particular group of organisms having a cell wall that includes the specific organism having a cell wall, and a sRNA species showing no expression only in a particular group of organisms having a cell wall that includes the specific organism having a cell wall.

In the identification method according to the present disclosure, the one or more sRNA species of which the presence state is to be detected preferably exhibit a specific expression profile in accordance with the organism having a cell wall.

As described above, in the determination method according to the present disclosure and the identification method according to the present disclosure, sRNAs can be allowed to escape into a water solvent outside a cell having a cell wall without performing denaturation of the cell membrane using, for example, strong alkaline treatment, guanidine treatment, phenol, an alcohol, or an ionic surfactant. Because of this, it is not necessary to use a denaturing agent, and treatment time with a denaturing agent is unnecessary. In addition, the water solvent to which sRNAs have escaped can be subjected, as it is, to a subsequent treatment (for example, a nucleic acid amplification treatment for sRNA detection).

### <Presence State>

The term "presence state" in the determination method according to the present disclosure and the identification method according to the present disclosure may refer simply to the presence or absence, or may refer to the abundance (including the case where the abundance is zero, i.e., absent). Accordingly, the expression "detecting the presence state" may represent obtaining binary information regarding the presence or absence, or may represent obtaining information regarding, in addition to the presence or absence, the abundance if present. The information regarding the abundance also inherently includes the information regarding the presence or absence. Similarly, the expression "determining the presence state" may represent making a binary determination regarding the presence or absence, or may represent making a determination regarding, in addition to the presence or absence, the abundance if present. Here, the abundance is not limited to an absolute abundance, but may be a relative abundance as compared to an item to be compared such as a negative control or a positive control.

### <Determination of Presence State>

The method used for detecting the presence state of sRNA species in the determination method according to the present disclosure and the identification method according to the present disclosure is not particularly limited. Examples of methods for detecting the presence state of sRNA species include hybridization with a labelled nucleic acid probe (including Northern blotting), and nucleic acid amplification. The nucleic acid amplification may be by any method that amplifies DNA or RNA, examples of which include amplification methods such as PCR (Polymerase Chain Reaction), RT-PCR (Reverse Transcription-PCR), LCR (Ligase Chain Reaction), SDA (Strand Displacement Amplification), NASBA (Nucleic Acid Sequence-based Amplification), TRC (Transcription Reverse-transcription Concerted Reaction), LAMP (Loop-mediated Isothermal Amplification), RT-LAMP (Reverse Transcription-LAMP), ICAN (Isothermal and Chimeric Primer-initiated Amplification of Nucleic Acids), RCA (Rolling Cycle Amplification), Smart Amp (Smart Amplification Process), TMA (Transcription-mediated Amplification), TAS (transcription Amplification System), and 3SR (Self-sustained Sequence Replication System). In the case of a method whereby RNAs cannot be directly amplified, sRNAs may be first subjected to reverse transcription into DNAs, and the resultant DNAs may be subjected to nucleic acid amplification. In an embodiment, the detection of the presence state of one or more sRNA species is performed using isothermal gene amplification or PCR.

The nucleic acid amplification used to detect the presence state of sRNA is preferably LAMP or RCA, both of which are isothermal gene amplification methods. A particular example of RCA is SATIC (termed signal amplification by ternary initiation complexes). Isothermal gene amplification is preferred in that it does not require the preparation of equipment for amplification (equipment to change the temperature in accordance with the temperature cycle). Isothermal gene amplification can be performed at a temperature in a temperature range of, for example, from 10°C to 40°C, and, therefore, isothermal gene amplification can be performed at room temperature. In the present disclosure, the room temperature may be a temperature in a range of from 20°C to 40°C, unless otherwise specified in, for example, the working examples.

The PCR mentioned above may be qPCR (quantitative PCR), and qPCR may be real-time PCR. A particular example of real-time PCR is a method using Taqman^{®} miRNA assay (Thermo Fisher Scientific, Inc.). When qPCR is used, it becomes easy to perform quantitative analysis of the presence state of a sRNA.

Reagents such as probes or primers used for detection of a sRNA (hereinafter also referred to as "sRNA detection reagents") may be added to the water solvent after completion of immersing, or may be allowed to be contained in the water solvent in advance before immersing. Allowing the water solvent to include the sRNA detection reagents in advance before immersing is preferable in that an addition procedure after completion of immersing is unnecessary. In a case in which a measurement sample that is a water solvent is used as a liquid sample, sRNA detection reagents may be added to the liquid sample. In a case in which there is a process of preparing a measurement sample that is a water solvent, sRNA detection reagents may be added during the preparation process.

Amplified nucleic acids can be detected by using an existing amplified nucleic acid detection method, such as: allowing a primer to carry a fluorescent dye; visually observing precipitates; using a nucleic acid staining dye; allowing the amplified nucleic acids to hybridize with a fluorescent probe; or subjecting the amplified nucleic acids to nucleic acid chromatography. The fluorescent probe may be arranged on a microarray. By using the microarray, it is possible to obtain information regarding the presence state of plural sRNA species at once.

For example, by using a TAQMAN^{®} probe, SYBR Green dye, or the like, the amount of nucleic acids amplified can be measured based on a fluorescent signal, and, based on the information obtained, the presence state of a sRNA can be revealed. This method is particularly effective in the case of carrying out qPCR.

As described above, nucleic acid amplification is not essential for sRNA detection, and a sRNA may be directly detected, without nucleic acid amplification, by hybridization between the sRNA and a fluorescent probe.

In the detection procedure such as nucleic acid amplification or hybridization with a probe, the full length of the sRNA may be amplified, and/or a probe that hybridizes with the full length of the sRNA may be used. However, it is not essential to amplify the full length of the sRNA and/or to use a probe that hybridizes with the full length of the sRNA. Nucleic acid amplification or hybridization may be performed such that the nucleic acid amplification or hybridization is targeted at a part of a sRNA, for example, about 10 to about 30 bases at the 3' end region of the sRNA.

The detection of the presence state of sRNA species may be performed concurrently and in parallel with the extraction of the sRNAs from a cell having a cell wall. For example, a sample potentially containing an organism having a cell wall may be directly immersed in or added to a reaction solution for PCR or isothermal gene amplification such as SATIC, and escape of sRNAs and detection of sRNAs can be performed concurrently. In the case of isothermal gene amplification, since isothermal gene amplification does not involve temperature cycles, nucleic acid amplification can be started at any time as long as the reagents required for nucleic acid amplification are present in the liquid sample.

### <Determination of Presence State of Organism Having Cell Wall>

The fact that a sRNA specific to an organism having a cell wall is detected in a measurement sample suggests that the organism is present in the measurement sample. From the information regarding the abundance of the sRNA specific to the organism having a cell wall in a measurement sample, it is also possible to obtain information regarding the abundance of the organism in the measurement sample. In the present disclosure, it has been found, for the first time, that an organism present can be identified based on the presence state of sRNA species.

The organism that expresses a sRNA of interest can be found, for example, as follows. Using the nucleic acid sequence of the sRNA as a query sequence, nucleic acid sequences from the National Center for Biotechnology Information (NCBI) database that are similar to the query sequence are subjected to comparison using Nucleotide BLAST (https://blast.ncbi.nlm.nih.gov/Blast.cgi). An organism that is indicated to contain, among the comparison results obtained, a sRNA having a nucleic acid sequence exhibiting 100% match with the sRNA used as the query sequence is considered as the organism that expresses the sRNA.

The identity of sRNA species extracted to the outside of a cell is unknown before the presence state of the sRNA species is detected. Even so, when a specific sRNA is detected outside the cell, a determination that an organism known to have the specific sRNA is present can be made.

An organism having a cell wall in the determination method according to the present disclosure refers to an organism having a cell wall to be detected, and may be any organism having a cell wall to be detected. By detecting the presence state of a sRNA species that is specifically expressed in the organism compared to other organisms, the presence state of the organism can be determined. The fact that a sRNA species that is specifically expressed in the organism is detected suggests that the organism is present, and the fact that the sRNA species is not detected suggests that the organism is not present. In the determination method according to the present disclosure, it is preferable to select a sRNA species exhibiting a specific expression profile in the organism as a sRNA species of which the presence state is to be detected, in accordance with the type of the organism of which the detection of the presence state thereof in a measurement sample is desired. The specific expression profile of a sRNA species may mean that the sRNA species is specifically expressed in the organism of which the detection of the presence state thereof is desired, or that the sRNA species is specifically non-expressed in the organism of which the detection of the presence state thereof is desired. It is preferable that at least one of the one or more sRNA species of which the presence state is to be detected is a sRNA species that is specifically expressed in the organism of which the detection of the presence state thereof is desired.

It is not always the case that one sRNA species is completely specific to one organism, and there may be cases in which there are plural organisms that express the same sRNA species. However, since the sRNA expression profile in one organism varies with each sRNA species, the range of organisms that have the possibility of being present can be narrowed based on the presence state of the plural sRNA species. Therefore, in the determination method according to the present disclosure, the presence state of an organism may be determined based on the presence state of plural sRNA species. In this determination, the expression state of a sRNA species that is specifically non-expressed in the organism can also be used. The fact that a sRNA species that is specifically non-expressed in the organism is detected or not detected does not suggest, by itself, the presence state of the organism. However, when the fact is considered in combination with the information regarding the presence state of a sRNA species that is specifically expressed in the organism, the range of candidate organisms that exhibit the expression profile of the sRNA species can be narrowed.

In the determination method according to the present disclosure, it is preferable to select, as one or more sRNA species of which the presence state is to be detected, one or more sRNA species that enables a particular organism of interest to be better distinguished from other organisms based on the expression profile of the sRNA species.

Nevertheless, in the determination method according to the present disclosure, it is not essential to limit the candidate organisms having a cell wall and having a possibility of being present to one organism, and it is sufficient to identify a group of candidates consisting of plural organisms. Therefore, the detection method according to the present disclosure may be a method whereby the presence state of plural organisms is determined, in which case, the detection method is not a method whereby the presence state of each of the plural organisms is individually determined, but is a method whereby the presence state of the plural organisms is collectively determined. In other words, the detection method may satisfactorily be a method whereby a determination that at least one of the plural organisms constituting the candidate group is present can be made, even if the method is incapable of determining which of the plural organisms is actually present.

That is, in the determination method according to the present disclosure, determining the presence state of an organism may include determining the gross presence state of two or more organisms. When the determination method according to the present disclosure includes determining the gross presence state of two or more organisms, determining the gross presence state of two or more organisms may include determining whether none of the two or more organisms is present or at least one of the two or more organisms is present. In a case in which it is determined that at least one of the two or more organisms is present, determining the gross presence state may further include determining the abundance of the at least one of the two or more organisms that is present.

In this case, even though complete determination of the presence state of a single organism of interest among two or more organisms is not possible, it is nonetheless possible to determine the possibility of the presence of the single organism of interest. Further tests may be performed based on this determination result, or procedures such as cleaning the analysis object from which the measurement sample has been obtained may be carried out based on the determination result. Accordingly, determining the gross presence state of two or more organisms may include determining the possibility of the presence of a particular organism included in the two or more organisms, and may also include determining the estimated abundance of the particular organism.

When the presence state of one or more sRNA species is detected, the abundance of the organism described above can also be determined by measuring the abundance of the sRNA species. The detection of the abundance of a sRNA species can be performed by methods commonly used in the art, such as a technique of measuring the fluorescence emission amount from a fluorescence-labeled probe that hybridizes to the sRNA species, a technique of measuring the fluorescence emission amount from a fluorescence-labeled primer used in nucleic acid amplification from the sRNA species, or a technique using qPCR.

### <Identification of Organism Having Cell Wall Present in Measurement Sample>

From the presence state of one or more sRNA species obtained from a liquid sample, it is possible to identify one or more organisms having a sRNA expression profile that is in agreement with the presence state of the sRNA species. The one or more sRNA species preferably exhibit a specific expression profile in accordance with the organism. Detecting the presence state of one or more sRNA species in a liquid sample may include exhaustively detecting the sRNA species present in the liquid sample, or may include measuring the presence state of one or more sRNA species that have been selected in advance in consideration of the types of organisms that are potentially present in the measurement sample.

It is not always the case that the specific presence state of one sRNA species is completely specific to a sRNA expression profile of one organism, and there may be cases in which there are plural organisms that have a sRNA expression profile consistent with the specific presence state of the sRNA. However, since the sRNA expression profile in each organism varies with each sRNA species, the range of organisms that have the possibility of being present can be narrowed based on the information regarding the presence state of each of the plural sRNA species. Therefore, in the identification method according to the present disclosure, the organism present in the sample may be identified based on the presence state of each of the plural sRNA species. Nevertheless, in the identification method according to the present disclosure, it is not essential to limit the candidate organisms present to one organism, and it is sufficient to identify a group of candidates consisting of plural organisms.

Therefore, the identification method according to the present disclosure may be a method whereby plural organisms are identified, in which case, the identification method is not a method whereby the presence of each of the plural organisms is individually detected, but is a method whereby the presence of the plural organisms is collectively detected. In other words, the identification method may satisfactorily be a method whereby a detection that at least one of the plural organisms is present can be made, even if the method is incapable of detecting which of the plural organisms is actually present.

That is, in the identification method according to the present disclosure, identifying the organism present in the measurement sample may include identifying that at least one of two or more candidate organisms is present. When identifying the organism identifies that at least one of the two or more organisms is present, identifying the organism may further include identifying the abundance of the at least one of the two or more candidate organisms that is present. In the identification method according to the present disclosure, when identifying the organism present in a measurement sample includes identifying that at least one of two or more candidate organisms is present, exact determination as to which of the two or more candidate organisms is present is not possible. Even so, further tests may be performed based on this identification result, or procedures such as cleaning the analysis object from which the measurement sample has been obtained (appropriate cleaning in accordance with the type of the candidate microorganism) may be carried out based on the identification result.

Accordingly, identifying that at least one of two or more candidate organisms is present may include identifying a particular organism included in the two or more candidate organisms, as an organism having the possibility of being present, and may also include identifying the estimated abundance of the particular organism.

When the presence state of one or more sRNA species is detected, the abundance of the organism described above can also be determined by measuring the abundance of the sRNA species. This is because the abundance of the sRNA species in the liquid sample is considered to reflect the abundance of the organism that expresses the sRNA species (in the simplest case, proportional to the amount of the organism expressing the sRNA species). The detection of the abundance of a sRNA species can be performed by methods commonly used in the art, such as a technique of measuring the fluorescence emission amount from a fluorescence-labeled probe that hybridizes to the sRNA species, a techniques of measuring the fluorescent emission amount from a fluorescence-labeled primer used in nucleic acid amplification from the sRNA species, or a technique using qPCR (for example, determining the Ct value in real-time PCR).

An example of the identification of an organism based on the presence state of one or more sRNA species in a liquid sample in the identification method according to the present disclosure will be described. EC-5p-36 (SEQ ID NO: 1;
5'-UGUGGGCACUCGAAGAUACGGAU-3', see Curr Microbiol.2013 Nov; 67(5): 609-13), which is a sRNA species, is commonly expressed in *Escherichia* bacteria, *Shigella* bacteria, *Salmonella* bacteria, and *Citrobacter* bacteria, according to a Nucleotide BLAST search. Therefore, when the presence of EC-5p-36 in a liquid sample is detected, the organism present can be identified to be at least one of an *Escherichia* bacterium, a *Shigella* bacterium, a *Salmonella* bacterium, or a *Citrobacter* bacterium. EC-3p-40, which is sRNA species, is commonly expressed in *Shigella* bacteria, *Salmonella* bacteria, *Escherichia* bacteria, and *Citrobacter* bacteria, and, in addition, *Klebsilla* bacteria, according to a Nucleotide BLAST search. Therefore, when the presence of EC-3p-40 (SEQ ID NO: 2;
5'-GUUGUGAGGUUAAGCGACU-3') in a liquid sample is detected, the organism present can be identified to be at least one of a *Escherichia* bacterium, a *Shigella* bacterium, a *Salmonella* bacterium, a *Citrobacter* bacterium, or a *Klebsilla* bacterium. In addition, identification may be carried out by combining these results. For example, based on the fact that EC-5p-36 is not expressed in *Klebsilla* bacteria, but EC-3p-40 is expressed in *Klebsilla* bacteria, a detection result that EC-3p-40 is present but EC-5p-36 is not present in the liquid sample allows the identification that the organism present is a *Klebsilla* bacterium.

The identification of the organism may be carried out based on the presence state of a sRNA species having a specific function. For example, 24B_1 (SEQ ID NO: 3; 5'-UAACGUUAAGUUGACUCGGG-3', see Scientific Reports volume 5, Article number: 10080 (2015)), which is a sRNA species associated with a toxin (verotoxins, also called ciguatoxins) found in enterohemorrhagic *E. coli* (such as 0-157), is commonly expressed in verotoxin (ciguatoxin)-producing bacteria, according to a Nucleotide blast search. Therefore, when the presence of 24B_1 in a liquid sample is detected, the organism present can be identified to be a bacterium having a verotoxin.

In the determination method according to the present disclosure, the presence state of EC-5p-36 may be detected to determine the gross presence state of *Escherichia* bacteria, *Shigella* bacteria, *Salmonella* bacteria, and *Citrobacter* bacteria (for example, none of these bacteria is present, or bacteria of at least one of these genera are present). In the determination method according to the present disclosure, the presence state of EC-3p-40 and EC-5p-36 may be detected to determine the presence state of *Klebsilla* bacteria.

In addition, EC-5p-79 (SEQ ID NO: 11; 5'-UUUGCUCUUUAAAAAUC-3') and EC-3p-393 (SEQ ID NO: 12; 5'-CUCGAAGAUACGGAUUCUUAAC-3'), which are sRNA species, are expressed in *Escherichia coli, Citrobacter freundii,* and *Salmonella gallinarum.* Based on the foregoing, examples of correspondence between a sRNA species and an organism include a combination of at least one selected from the group consisting of EC-5p-36, EC-3p-40, EC-5p-79, and EC-3p-393 and at least one selected from the group consisting of *Escherichia coli, Citrobacter freundii,* and *Salmonella gallinarum,* a combination of fox_milRNA_5 having the nucleotide sequence of SEQ ID NO: 10 and *Fusarium oxysporum,* a combination of miR156 having the nucleotide sequence of SEQ ID NO: 4 and black pine, and a combination of miR716b having the nucleotide sequence of SEQ ID NO: 5 and *Saccharomyces cerevisiae.*

The sRNA species to be detected is preferably at least one selected from the group consisting of EC-5p-36, EC-3p-40, EC-5p-79, EC-3p-393, fox_milRNA_5, miR156, and miR716b, which are used in the tests described in the working examples.

Although a few examples of sRNA species have been mentioned above, the determination method according to the present disclosure and the identification method according to the present disclosure can also be performed, in a similar manner, in the case of detecting other sRNA species. The correspondence between the sRNA species and the organism required for the determination or the identification can be easily obtained from the database described above.

In consideration of the embodiments described above, the following embodiments are also provided according to the present disclosure. The method of detecting a detection target organism according to the present disclosure includes:
immersing a detection target organism contained in a measurement sample in a water solvent, to prepare a liquid sample in which one or more sRNA species specific to the detection target organism have escaped into the water solvent; and
detecting the one or more sRNA species in the liquid sample.

The method used for immersing the detection target organism in a water solvent is not particularly restricted as long as the detection target organism contacts the water solvent, and can be performed in the same manner as that in the aforementioned immersion of a measurement sample or preparation of a measurement sample that is a water solvent in the determination method according to the present disclosure and the identification method according to the present disclosure. As long as the one or more sRNA species specific to the detection target organism include at least one sRNA species that is specifically expressed in the detection target organism, the other sRNA species in the one or more sRNA species may be specifically non-expressed in the detection target organism.

As described above, in the determination method according to the present disclosure and the identification method according to the present disclosure, sRNAs can be allowed to escape from a cell having a cell wall into a water solvent in a simple manner without performing a membrane denature procedure with a strong denaturing agent. Further, by detecting the presence state of the sRNA species that have escaped (for example, by detecting the sRNA species by nucleic acid amplification), the presence state of a specific organism of interest can be determined, and an organism present in the measurement sample can be identified, with reduced burden as compared to the method of extracting 16S rRNA by denaturing the membrane. In particular, in a case in which isothermal gene amplification (for example, RCA), in which the reaction proceeds at room temperature, is used as the nucleic acid amplification method, the presence state of an organism can be determined or an organism present in the measurement sample can be identified in a simple manner, while using no special equipment (for example, equipment to execute temperature cycles) from the beginning to the end.

According to the determination method according to the present disclosure and the identification method according to the present disclosure, there is no need to culture organisms that are potentially contained in the measurement sample, in contrast to culture methods. Therefore, although determination or identification in a short time is possible, high sensitivity can also be achieved by using, for example, nucleic acid amplification. Furthermore, the determination method according to the present disclosure and the identification method according to the present disclosure can be applied to organisms, such as bacteria, that are difficult to culture. In addition, unlike culture methods, the determination method according to the present disclosure and the identification method according to the present disclosure require only a small sample amount, and disposal of materials used for treatment is easy. The determination method according to the present disclosure and the identification method according to the present disclosure enable determination of the presence state of an organism and identification of an organism contained in a measurement sample with a simpler operation than conventional genetic methods, while maintaining the advantages of the genetic methods.

The determination method according to the present disclosure and the identification method according to the present disclosure can be used, for example, for simple microorganism tests at sites at which hygiene control is required (for example, food production, medical care, welfare, and household). Examples thereof include: hygiene control of products by testing for putrefactive and deteriorating organisms in food manufacturing processes; rapid identification of the cause at food poisoning accidents; prevention of secondary infection by detection of food poisoning bacteria in beds and waiting rooms of medical facilities; prevention of secondary infection with food poisoning bacteria in welfare facilities for children and the elderly; and prevention of secondary infection by detection of food poisoning bacteria in a case in which a food poisoning patient stays at home.

### EXAMPLES

Embodiments will be further described with reference to examples below. However, the present disclosure is not limited to the following examples in any way. Unless otherwise specified, the term "%" indicating the amount of a component contained in a composition in the examples is based on mass. The "room temperature" in the following examples was approximately 30°C.

### <sRNA Quantification Method by Real-time PCR>

In Examples 1 to 3, 7 to 9, and 11, sRNA species of which the presence state was to be determined were quantified by the method described below.

Specifically, sRNA species in the reaction solution in each of Examples 1 to 3, 7 to 9, and 11 was quantified by a real-time PCR method. Reverse transcription was carried out in accordance with the manufacturer's instructions using a quantification reagent (Taqman^{®} microRNA Assays, manufactured by Applied Biosystems), which was prepared by custom synthesis suited for the sRNA species to be quantified, and a reverse transcriptase (Taqman^{®} microRNA RT Kit, manufactured by Applied Biosystems), thereby obtaining a reverse transcription solution containing DNA formed by the reverse transcription. Specifically, 1 µL of RNA sample, 1.5 µ of 10× RT buffer, 0.15 µL of dNTP mix, 0.19 µL of RNase inhibitor, the first solution of the custom-synthesized Taqman assays in an amount to provide a final concentration of 1 × (for example, 0.75 µL in the case of 20×Taqman assays), and 1 µL of Multiscribe RT enzyme were used in a single reaction in the reverse transcription, and the liquid volume was adjusted to 15 µL with pure water. The temperature profile of the reverse transcription included the steps of (1) 16°C for 30 min, followed by (2) 42°C for 30 min, and (3) 85°C for 5 min.

Then, a real-time PCR reaction was allowed to proceed using the reverse transcription solution obtained. A reaction solution was prepared in accordance with the manufacturer's instructions using a quantification reagent (TAQMAN^{®}, microRNA Assays, manufactured by Applied Biosystems), which was prepared by custom synthesis suited for the sRNA species to be quantified, and real-time PCR Master Mix (TAQMAN^{®} Universal PCR Master Mix II with UNG, manufactured by Applied Biosystems). Specifically, a reaction solution was prepared using 2 µL of the reverse transcription solution, 10 µL of Universal PCR Master Mix II with UNG, and the second solution of the custom-synthesized Taqman assays in an amount to provide a final concentration of 1 × (for example, 1 µL in the case of 20× TAQMAN assays), with the liquid volume adjusted to 20 µL with pure water. The prepared reaction solution was subjected to real-time PCR using a STEPONEPLUS^{®} Real-Time PCR System (manufactured by Applied Biosystems) with a temperature profile including 40 cycles of 95°C for 10 min, then 95°C for 15 sec, and 60°C for 1 min. In real-time PCR, using the STEPONEPLUS^{®} Real-Time PCR System (manufactured by Applied Biosystems), Ct values were calculated by automatic calculation of the STEPONEPLUS software under the conditions of reagents = "Taqman reagents" and ramp speed = "Standard" (the settings other than these were default settings). In the case of quantification by comparison with standard samples, the sRNA sequence to be quantified was synthesized (RNA primer synthesis by Eurofins Genomics; HPLC purification grade), and a dilution series was prepared in a range of from 10⁻¹⁰ M to 10⁻¹⁵ M. Real-time PCR was also performed with respect to the dilution series concurrently in parallel with the measurement of the samples, and the sRNA amount was quantified by comparing the Ct values.

### (Example 1) Simple Extraction of sRNA Contained in Escherichia coli (EC-5p-36)

With respect to EC-5p-36 as the target, which is a sRNA species contained in *Escherichia coli* W3110 (hereinafter simply referred to as "*E. coli* W3110"), simple extraction from *E. coli* W3110 was attempted.

A colony of *E. coli* W3110 was suspended in 100 µL of pure water to obtain a 90 mass% *E. coli* W3110 microbial cell suspension. The following three samples were prepared from the 90 mass% *E. coli* W3110 microbial cell suspension.

Sample 1): RNase inhibitor (manufactured by Toyobo Co., Ltd.) was added to the 90 mass% *E. coli* W3110 microbial cell suspension such that the final concentration of the RNase inhibitor became 0.4 U/µL, whereby 10 µL of a reaction solution was prepared. The reaction solution was left to stand at room temperature for 4 hours, to obtain Sample 1.

Sample 2): The 90 mass% *E. coli* W3110 microbial cell suspension was immediately purified using a MIRNEASY kit (a cell lysis reagent that contains phenol and causes cell membrane denaturation; manufactured by QIAGEN N.V.), and total sRNAs were extracted to obtain Sample 2.

Further, 10 µL of pure water was used, as it is, as Sample 0, which serves as a reference.

With respect to Samples 0 to 2, EC-5p-36 was quantified by real-time PCR. The sRNA amounts in Samples 0 to 2 obtained by the quantification were each divided by the sRNA amount in Sample 2, and the obtained values were regarded as the relative extraction ratios (Table 1).

**Table. 1: Relative Extraction Ratio of EC-5p-36**

| Sample No. | Components of Reaction Solution or Treatment | Relative Extraction Ratio of EC-5p-36 |
|---|---|---|
| 0 | Water only | 0% |
| 1 | Water and *E. coli* W3110 | 8% |
| 2 (reference) | Phenol treatment on water and *E*. *coli* W3110 | (100%) |

As demonstrated by the results in Table 1, it was found that sRNA can be extracted from the microbial cells in a simple manner and can be measured. The sRNA could be extracted even with pure water alone.

### (Example 2) Simple Extraction of sRNA Contained in Black Pine Pollen (miR156)

With respect to miR156 as the target, which is a sRNA species contained in black pine pollen (SEQ ID NO: 4; 5'-CAGAAGAUAGAGAGCACAUC-3'; see http://www.mirbase.org/;pta-miR156a), simple extraction from black pine pollen was attempted.

10 mg of black pine pollen (manufactured by Biostir Inc.) was suspended in 1 mL of pure water and left to stand at room temperature for 1 hour. Then, miR156 was quantified by real-time PCR. Whether or not the sRNA species (miR156) could be detected was compared using RNase-free water as a negative control, and water containing 1 nM synthetic miR156 (SEQ ID NO: 4; synthesized by Eurofins Genomics) as a positive control. As a result, it was confirmed that sRNA can be extracted from black pine pollen even by simply suspending the black pine pollen in pure water.

**Table 2: Detection of miR156**

| Components | Detection of miR156 (real-time PCR) |
|---|---|
| Water only | not detected |
| Water and synthetic miR156 | detected |
| Water and black pine pollen | detected |

(Example 3) Simple Extraction of sRNA Contained in Yeast (miR716b)

With respect to miR716b as the target, which is a sRNA species contained in Saccharomyces cerevisiae (hereinafter simply referred to as "S. cerevisiae") (SEQ ID NO: 5; 5'-GAGAUCUUGGUGGUAGUAGCAAAUA-3'; see Sci. Rep., 2015,5,7763), simple extraction from *S*. *cerevisiae* was attempted.

*S. cerevisiae* was cultured on an LB plate. Ten milligrams of *S*. *cerevisiae* was scraped off, suspended in 1 mL of pure water, and left to stand at room temperature for 1 hour. After the standing, extraction of miR716b was attempted using the real-time PCR. Whether or not the sRNA species could be detected was compared using RNase-free water as a negative control, and 1 nM synthetic miR716b (SEQ ID NO: 5; synthesized by Eurofins Genomics K.K.) as a positive control. As a result, it was confirmed that sRNA from S. cerevisiae can be extracted even by simply suspending in pure water, and that the amount of extracted sRNA can be detected quantitatively.

**Table 3: Detection of miR716b**

| Components | Detection of miR716b (real-time PCR) |
|---|---|
| Water only | not detected |
| Water and synthetic miR716b | detected |
| Water and *S*. *cerevisiae* | detected |

### (Example 4) Detection of Escherichia coli-derived sRNA (EC-5p-36) by Isothermal Gene Amplification

With respect to EC-5p-36 as the target, which is a sRNA species contained in *Escherichia coli,* detection of the sRNA was attempted by isothermal gene amplification (SATIC method; Anal Chem. 2016 Jul 19; 88 (14): 7137-44)) of the sRNA that had been obtained by simple extraction from *E. coli* with a water solvent.

### <Synthesis of Cyclized T1B-EC-5p-36>

First, a primer, which is a synthetic single-stranded DNA having the following nucleotide sequence, was obtained from Eurofins Genomics based on our order for the synthesis thereof.

Primer sequence (SEQ ID NO: 6): 5'-CCCCAAAAAATCCGTATCTTCGAGTGCCCACAAAAAAGAAGCTGTTGTATTGT TGTCGAAGAAGAAAAGT-3' (5'-phosphorylated and HPLC-purified)

Then, the synthetic single-stranded DNA described above was cyclized using a CIRCLIGASE II ssDNA Ligation kit (Lucigen Corporation) in accordance with the manual. Specifically, 15 µL of RNase-free water (QIAGEN K.K.), 1 µL of the 10 pmol/µL synthetic single-stranded DNA described above, 2 µL of CircLigase II 10× Reaction Buffer, 1 µL of 50 mM MnCl₂, and 1 µL of CircLigase II ssDNA Ligase were added into a PCR tube. After mixing them, the PCR tube was incubated on a thermal cycler at 60°C for 1 hour and at 80°C for 10 minutes, to obtain cyclized DNA (hereinafter referred to as "cyclized T1B-EC-5p-36") at 500 nM. The resultant solution was diluted 5-fold with RNase-free water to obtain cyclized T1B-EC-5p-36 at 100 nM.

### <Synthesis of Cyclized T2>

First, a primer which is a synthetic single-stranded DNA having the following nucleotide sequence, was obtained from Eurofins Genomics based on our order for the synthesis thereof.

Primer sequence (SEQ ID NO: 7): 5'-CCCAACCCTACCCACCCTCAAGAAAAAAAAGTGATAATTGTTGTCGAAGAAGA AAAAAAATT-3' (5'-phosphorylated and HPLC-purified)

Then, using a CIRCLIGASE II ssDNA Ligation kit (Lucigen Corporation), the same procedures as those in the synthesis of cyclized T1B-EC-5p-36 were carried out except that the primer of SEQ ID NO: 7 (T2) was used in place of the primer of SEQ ID NO: 6 (T1B-EC-5p-36), and cyclized DNA (hereinafter referred to as "cyclized T2") at 500nM was obtained. The resultant solution was diluted 1.25-fold with RNase-free water to obtain cyclized T2 at 400 nM.

### <Synthesis of Primer P2>

First, a synthetic primer having the following nucleotide sequence was obtained from Eurofins Genomics K.K based on our order for the synthesis thereof.

Primer sequence (SEQ ID NO: 8): 5'-GAAGCTGTTGTTATCACT-3' (free of modification; HPLC-purified)

The primer was diluted with RNase-free water to prepare primer P2 at 480 nM.

### <Detection of E. coli-derived sRNA by Isothermal Gene Amplification>

Detection of an *E*. coli-derived sRNA species was attempted using ϕ29 DNA polymerase (a kit manufactured by New England BioLabs, Inc.). Specifically, 5.8 µL of RNase-free water, 2 µL of 480 µM primer P2, 2 µL of 100 nM cyclized T1B-EC-5p-36, 2 µL of 400 nM cyclized T2, 2 µL of dNTP Mix containing each NTP at 10 mM, 2 µL of 10× ϕ29 DNA polymerase Reaction buffer, 0.2 µL of BSA included in the kit, and 2 µL of cp29 DNA polymerase were mixed in a PCR tube for each sample, thereby preparing a premix. A *E*. *coli* W3110 colony cultured on LB medium was suspended in 1mL of RNase-free water, and 2 µL of the suspension was added to the premix, to obtain a sample. In addition, 2 µL of 10 nM synthetic EC-5p-36 (SEQ ID NO: 1; synthesized by Eurofins Genomics) was added to the premix, to obtain a positive control. Further, 2 µL of RNase-free water was added to the premix, to obtain a negative control.

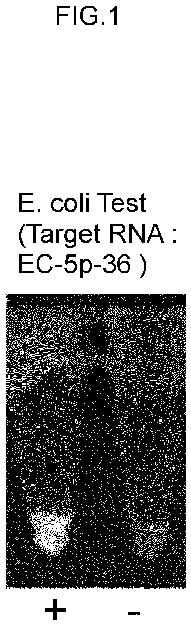

The sample, the positive control, and the negative control were incubated by being left to stand at 37°C for 16 hours. Then, 2 µL of a 100-fold dilution of SYBR Gold Nucleic Acid Gel Stain (Invitrogen), which is a reagent exhibiting an enhanced fluorescence intensity upon binding to nucleic acid, was added to the solution after incubation, followed by mixing. The reaction solution thus obtained was irradiated with black light (365 nm) and fluorescence emission was observed. As a result, strong emission of yellow-green fluorescence was observed in the sample and the positive control, while only weak emission of yellow fluorescence derived from SYBR Gold Nucleic Acid Gel Stain was observed in the negative control. Therefore, it was demonstrated that, in the case of the sample, the sRNA species, which was extracted from *E. coli* W3110 with a water solvent, was amplified by isothermal gene amplification and the amplified product could be detected based on fluorescence. A comparison between the fluorescence from the negative control and the fluorescence from the sample is indicated in Fig. 1. In Fig. 1, "+" represents the sample, and "-" represents the negative control.

### (Example 5) Detection of Black Pine Pollen-derived sRNA by Isothermal Gene Amplification

Detection of a black pine pollen-derived sRNA species was attempted by carrying out the same procedures as those in the method of detecting an *E*. *coli*-derived sRNA species by isothermal gene amplification in Example 4.

### <Synthesis of Cyclized T1B-miR156>

T1B-miR156 was prepared in the same manner as that in the preparation of T1B-EC-5p-36 in Example 4. First, a primer, which is a synthetic single-stranded DNA having the following nucleotide sequence, was obtained from Eurofins Genomics based on our order for the synthesis thereof.

Primer sequence (SEQ ID NO: 9): 5'-CCCCAAAAAGGAGCGATGTGCTCTCTATCTTCTGAAAAGAAGCTGTTGTATTGT TGTCGAAGAAGAAAAGT-3' (5'-phosphorylated and HPLC-purified)

The same procedures were carried out as those in Example 4 except that a primer of SEQ ID NO: 9 was used in place of the primer of SEQ ID NO: 6, to obtain cyclized DNA (hereinafter referred to as "cyclized T1B-miR156") at 100 nM.

### <Synthesis of Cyclized T2>

A cyclized T2 was obtained using the same method as that used in Example 4.

### <Synthesis of Primer P2>

A primer P2 was obtained using the same method as that used in Example 4.

### <Detection of Black Pine Pollen-derived sRNA by Isothermal Gene Amplification>

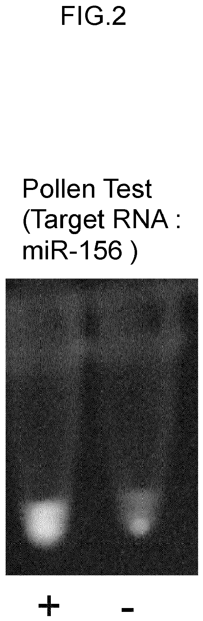

Detection of black pine pollen-derived sRNA was attempted in the same manner as that in Example 4. Specifically, the same experiment as that in Example 4 was performed, except that cyclized T1B-miR156 was used in place of cyclized T1B-EC-5p-36 used in Example 4, that synthetic miR-156 (SEQ ID NO: 4: synthesized by Eurofins Genomics) was used in place of synthetic EC-5p-36, and that the water suspension of black pine pollen was used in place of the water suspension of the cultured colony of *E. coli* W3110. As a result, strong emission of yellow-green fluorescence was observed in the sample (water suspension of black pine pollen) and in the positive control (synthetic miR-156), while only weak emission of yellow fluorescence derived from SYBR Gold Nucleic Acid Gel Stain was observed in the negative control (RNase-free water). Therefore, it was demonstrated that, in the case of the sample, the sRNA species, which was extracted from black pine pollen with a water solvent, was amplified by isothermal gene amplification, and the amplified product could be detected based on fluorescence. A comparison between the fluorescence from the negative control and the fluorescence from the sample is indicated in Fig. 2. In Fig. 2, "+" represents the sample, and "-" represents the negative control.

### (Example 6) Extraction of sRNA from Fusarium oxysporum

Fusarium oxysporum IFO5942 was cultured on an LB plate for 3 days. Then, 1 mg of this microorganism were scraped off and suspended in 1 mL of water containing 1% RNase inhibitor (Toyobo Co., Ltd.). Immediately after suspending or after the suspension was left to stand at 25°C for 16 hours, the suspension was filtered through a 100K Amicon Ultra 0.5 filter (Merck Millipore), and 10 µL of the filtrate was diluted with 90 µL of sterile water. After the solution was left to stand at 25°C for 16 hours, 10 µL of 1000-fold diluted SYBR Gold Nucleic Acid Gel Stain (manufactured by Thermo Fisher Scientific, Inc.) was added thereto. The fluorescence of the suspension (excitation wavelength: 495 nm, emission wavelength: 540 nm) was measured on a fluorescence plate reader (SPECTRAMAX 3i, manufactured by Molecular Probes). The fluorescence intensity immediately after suspending was 3.4 × 10⁶ RFU, while the fluorescence intensity increased to 4.2 × 10⁶ RFU after 2 hours. Thus, it was confirmed that the sRNA was released from *Fusarium* by suspending *Fusarium* in water, and that quantitative measurement of the released sRNA is possible.

### (Example 7) Detection of E. coli-derived sRNA (EC-5p-36) and Fusarium Oxysporum-derived sRNA (fox_milRNA_5)

*Fusarium oxysporum* IFO5942 was cultured on an LB plate at 25°C for 3 days.

Then, this microorganism was scraped off, suspended in 1 mL of pure water, and left to stand at room temperature for 1 hour. After the standing, the *Fusarium oxysporum* microbial cell suspension was diluted with pure water to an OD600 value of 0.01, thereby obtaining an OD0.01 *Fusarium* microbial cell suspension.

*E. coli* W3110 was cultured on an LB plate at 37°C for 1 day. Then, a colony was scraped off, suspended in 100 µL of pure water, and left to stand at room temperature for 1 hour. After the standing, the *E. coli* W3110 microbial cell suspension was diluted with pure water into an OD600 value of 0.1, thereby obtaining an OD0.1 *E. coli* W3110 microbial cell suspension.

Using the microbial cell suspensions obtained, quantification of EC-5p-36 and fox_milRNA_5 by real-time PCR was performed. EC-5p-36 sRNA (SEQ ID NO: 1) is a sRNA species that is expressed in *E. coli* W3110, but not expressed in *Fusarium oxysporum* IFO5942. Conversely, fox_milRNA_5 sRNA (SEQ ID NO: 10; 5'-UCCGGUAUGGUGUAGUGGC-3', see PLoS One. 2014 Aug 20; 9(8): e104956.) is a sRNA species that is not expressed in *E. coli* W3110, but expressed in *Fusarium oxysporum* IFO5942.

Real-time PCR was performed according to <sRNA Quantification Method by Real-time PCR> described above, using 1 µL sample of OD0.01 Fusarium microbial cell suspension and 1 µL sample of OD0.1 *E. coli* W3110 microbial cell suspension. In each of a case in which the detection target was fox_milRNA_5 and a case in which the detection target was EC-5p-36, a TAQMAN^{®} Assay primer having a nucleotide sequence suited for the detection target was used. Table 4 indicates the Ct value results obtained by the STEPONEPLUS^{®} Real-Time PCR System (Applied Biosystems).

**Table 4**

| Detection Target | Microbial Cell Suspension | TAQMAN Detection Value (Ct value) |
|---|---|---|
| fox_milRNA_5 | OD0.01 *Fusarium* | 30.1404 |
| | OD0.1 *E. coli* W3110 | 34.4908 |
| EC-5p-36 | OD0.01 *Fusarium* | 34.6899 |
| | OD0.1 *E. coli* W3110 | 29.9090 |

As demonstrated by the results in Table 4, it was found that fox_milRNA_5 can be specifically detected from *Fusarium* and that EC-5p-36 can be specifically detected from *E*. *coli* W3110. It was also demonstrated that quantitative detection of the sRNA by real-time PCR is possible.

### (Example 8) Study Concerning Temperature Conditions in Extraction Process

*Escherichia coli* DH5α was inoculated and cultured on an LB plate, and a resulting colony was suspended in 2 mL of LB medium and cultured with shaking at 37°C and 180 rpm for 24 hours. Then, 40 µL of the culture liquid was collected, inoculated in 4 mL of LB medium, and cultured with shaking at 37°C and 180 rpm for 4 hours. Then, the culture liquid was centrifuged at 3,000 G for 5 minutes, the supernatant was removed by aspiration, and then sterile water was added. At this time, OD600 was measured and adjusted to OD = 0.1 with sterile water, thereby preparing an *E. coli* suspension.

Three 500 µL samples were prepared from the prepared *E. coli* suspension and left for 1 hour at 5°C, 25°C, and 37°C, respectively. Thereafter, the *E. coli* suspensions were filtered through a 0.22 µm filter.

Reverse transcription and real-time PCR targeted for each of EC-5p-36 and 16S rRNA in each filtrate were performed. 1 µL of the filtrate was collected as an RNA sample. Real-time PCR for EC-5p-36 was performed according to <sRNA Quantification Method by Real-time PCR> described above, and real-time PCR for 16S rRNA was performed according to <16S rRNA Quantification Method by Real-time PCR> described below. Table 5 indicates the Ct value results obtained by real-time PCR. It was found that the extracted 16S rRNA exhibited only a low concentration in each case. In contrast, EC-5p-36 was extracted in an amount sufficient for determination of its presence, at each of the temperatures of 10°C, 25°C, and 37°C. In addition, the Ct value decreased as the temperature at extraction (immersion) increased, from which the tendency that the sRNA is more easily extracted as the temperature increases can be observed. As discussed above, it was found that EC-5p-36, having a relatively small number of bases (23 bases), exhibits a small Ct value and can be detected in a stable manner. In contrast, it was found that 16S rRNA, having a relatively large number of bases (about 1,500 bases), exhibits a large Ct value and is difficult to detect.

**Table 5**

| Treatment Temperature | EC-5p-36 Ct value | 16S rRNA Ct value |
|---|---|---|
| 37°C | 24.8 | 33.2 |
| 25°C | 27.0 | 34.4 |
| 10°C | 27.1 | 33.2 |

### The method used for quantifying 16S rRNA by real-time PCR is described below. <16S rRNA Quantification Method by Real-time PCR>

16S rRNA of which the presence state is to be determined was quantified by the method described below. Using 1 µL of RNA sample, preparation was carried out to adjust the liquid volume to 10 µL. The 10 µl reaction solution was subjected to reverse transcription. The reaction solution contained 100 nM primer (SEQ ID NO: 13: CCGGGAACGTATTCACC), 0.4% Moloney Murine Leukemia Virus Reverse Transcriptase (manufactured by Promega Corporation), 20% 5X Reaction Buffer, dNTPs at 0.4 mM each, 0.1% RNase inhibitor (manufactured by Toyobo Co., Ltd.), and 10% of the liquid extract (RNA sample). The temperature profile of the reverse transcription included steps consisting of (1) 16°C for 30 min, followed by (2) 42°C for 20 min, and (3) 85°C for 5 min.

Then, real-time PCR was carried out using the reverse transcription solution obtained. Using 2 µL of the reverse transcription solution, it was adjusted to a volume of 20 µL. The PCR reaction solution used contained 300 nM primer 1 (SEQ ID NO: 14: AGAGTTTGATCATGGCTCAG), 300 nM primer 2 (SEQ ID NO: 15: CCGGGAACGTATTCACC), 200 µM dNTPs (CLEANAMP^{™} Hot Start dNTP Mix, obtained from Sigma-Aldrich, USA) (note: purification by filtration had been performed in advance using an Amicon Ultra 50 K centrifugal filter available from Merck Millipore), 50 mM KCl, 2.25 mM MgCl₂, 10 mM Tris-HCl (pH8.3), 1× EvaGreen (obtained from Biotium Inc., CA, USA), 0.05 units/µL eukaryote-made thermostable DNA polymerase (see J Clin Microbiol. 2011 49(9) 3316-3320), and 10% of the reverse transcription solution. The temperature profile of the real-time PCR included a denaturation step at 95°C for 10 min, followed by 40 cycles of 94°C for 10 seconds, 65°C for 20 seconds, 72°C for 30 seconds, and 85°C for 10 seconds. Using the StepOnePlus^{®} Real-Time PCR System (manufactured by Applied Biosystems), Ct values were calculated by automatic calculation of the STEPONEPLUS software under the conditions of reagents = "SYBR Green reagents" and ramp speed = "Standard" (the settings other than these were default setting).

### (Example 9) Detection of Bacteria Belonging to Enterobacteriaceae.

Each of *Escherichia. coli*, *Citrobacter ƒreundii*, and *Salmonella gallinarum*, which are bacteria belonging to *Enterobacteriaceae*, was inoculated and cultured on a LB plate, and a resulting colony was suspended in 2 mL of LB medium and cultured with shaking at 37°C and 180 rpm for 24 hours. Then, 40 µL of the culture liquid was collected and inoculated in 4 mL of LB medium, and cultured with shaking at 37°C and 180 rpm for 24 hours. Then, the culture liquid was centrifuged at 3,000 G for 5 minutes, the supernatant was removed by aspiration, and then sterile water was added. At this time, OD600 was measured and adjusted to OD = 1 with sterile water, to prepare a microbial cell suspension.

Three 500 µL samples were prepared from the prepared microbial cell suspension and left for 1 hour at 5°C, 25°C, and 37°C, respectively. Thereafter, the microbial cell suspensions were filtered through a 0.22 µm filter.

Reverse transcription and real-time PCR targeted for each of sRNA species (EC-5p-36, EC-3p-40, and EC-5p-79) in each filtrate were performed. Real-time PCR was performed according to <sRNA Quantification Method by Real-time PCR> described above using 1 µL of the filtrate as a sample. Table 6 indicates the Ct value results obtained by the Real-Time PCR System (Applied Biosystems). In all cases, the Ct values were 28 or less, so that the presence of a bacterium of interest belonging to *Enterobacteriaceae* was detected.

**Table 6**

| sRNA Name | Nucleotide Sequence | Bacterium Contained in Sample | Ct Value: ≤ 28 |
|---|---|---|---|
| EC-5p-36 | UGUGGGCACUCGAAGAUACGGAU | *Escherichia coli* | Yes |
| | | *Citrobacter freundii* | Yes |
| | | *Salmonella gallinarum* | Yes |
| EC-3p-40 | GUUGUGAGGUUAAGCGACU | *Escherichia coli* | Yes |
| | | *Citrobacter freundii* | Yes |
| | | *Salmonella gallinarum* | Yes |
| EC-5p-79 | UUUGCUCUUUAAAAAUC | *Escherichia coli* | Yes |
| | | *Citrobacter freundii* | Yes |
| | | *Salmonella gallinarum* | Yes |
| EC-3p-393 | CUCGAAGAUACGGAUUCUUAAC | *Escherichia coli* | Yes |
| | | *Citrobacter freundii* | Yes |
| | | *Salmonella gallinarum* | Yes |

### (Example 10) Electrophoresis of Nucleic Acid Liquid Extract

*Escherichia coli* W3110 was inoculated and cultured on an LB plate, and a resulting colony was suspended in 2 mL of LB medium and cultured with shaking at 37°C and 180 rpm for 24 hours. Then, 100 µL of the culture liquid was collected and inoculated in 10 mL of LB medium, and cultured with shaking at 37°C and 180 rpm for 4 hours. Then, the culture liquid was centrifuged at 3,000 G for 5 minutes, the supernatant was removed by aspiration, and then sterile water was added. At this time, OD600 was measured and adjusted to OD = 1 with sterile water, to prepare a microbial cell suspension.

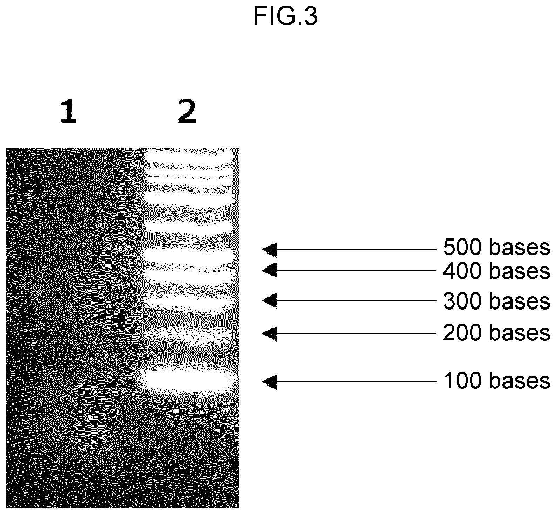

500 µL of the microbial cell suspension was left to stand at 37°C for 1 hour. Thereafter, the suspension was centrifuged at 3,000 G for 5 minutes and the supernatant was filtered through a 0.22 µm filter. 10 µl of the filtrate was mixed with 2 µL of 6x Loading buffer (manufactured by Takara Bio Inc.), added to a 2% agarose gel (LO3, Takara Bio Inc.), and electrophoresed in TAE (Tris-acetate with EDTA) buffer. The results are indicated in Fig. 3. Gene ladder wide 1 (manufactured by Nippon Gene Co., Ltd.) was used as the Ladder (lane 2 on the right). As a fluorescent substance to stain nucleic acids, 0.01% SYBR Green II (manufactured by Takara Bio Inc.) was used. Lane 1 on the left is the lane in which the filtrate was loaded.

As a result of the electrophoresis, fluorescence was observed in the region corresponding to a length of 500 bases or less, especially in the region corresponding to a length of 100 bases or less. Fluorescence depends on the concentration of the substance. Since the number of molecules having a smaller molecular weight would be higher if the fluorescence is the same, it was demonstrated that the concentration (number of molecules) of nucleic acids having a length of 100 bases or less was particularly high.

### (Example 11) Detection of Airborne Microorganism

*Escherichia coli* DH5α was inoculated and cultured on an LB plate, and a resulting colony was suspended in 2 mL of LB medium and cultured with shaking at 37°C and 180 rpm for 24 hours. Then, 100 µL of the culture liquid was collected and inoculated in 10 mL of LB medium, and cultured with shaking at 37°C and 180 rpm for 4 hours. Then, the culture liquid was centrifuged at 3,000 G for 5 minutes, the supernatant was removed by aspiration, and then sterile water was added. At this time, OD600 was measured and adjusted to OD = 1 with sterile water to prepare *E. coli* suspension. The obtained *E. coli* suspension was added into a glass sprayer having a volume of 30 mL.

An empty Petri dish (9 cm in diameter) for collecting airborne bacteria and a Petri dish (9 cm in diameter) containing LB solid medium as a positive control were placed on a tabletop, and were sprayed once with the *E. coli* suspension from 30 cm above using the glass sprayer. The Petri dish containing the LB solid medium was covered with a lid, placed in an incubator set at 37°C, and cultured for one day. Sterile water in an amount of 500 µL was added into the empty Petri dish, and the sterile water was fully spread over the Petri dish using a bacteria spreader. Then, the liquid in the Petri dish was collected and left to stand at 37°C for 1 hour. Using the collected liquid from the empty Petri dish, the abundance of EC-5p-36 was measured by real-time PCR according to <sRNA Quantification Method by Real-time PCR> described above, as a result of which the Ct value was found to be 28 or less. Thus, the presence of EC-5p-36 was confirmed. Separately, real-time PCR was directly performed on sterile water as a negative control to measure the abundance of EC-5p-36, and the Ct value was found to be 33 or more, which indicates absence of EC-5p-36. Further, in the LB Petri dish as the positive control, colony formation was observed.

The results of Example 11 demonstrate that the determination method according to the present disclosure and the identification method according to the present disclosure are also applicable to measurement samples obtained by collecting airborne microorganism.

The disclosure of Japanese Patent Application No. 2019-38910, filed March 4, 2019, is incorporated herein by reference in its entirety.

All publications, patent applications, and technical standards mentioned in this specification are herein incorporated by reference to the same extent as if each individual publication, patent application, and technical standard was specifically and individually indicated to be incorporated by reference.

## Claims

1. A method of determining a presence state of an organism having a cell wall, the method comprising:
preparing a liquid sample by immersing a measurement sample in a water solvent, or preparing a measurement sample that is a water solvent as a liquid sample; and
detecting a presence state of one or more sRNA species in the liquid sample,
wherein a presence state of an organism having a cell wall is determined based on the presence state of the one or more sRNA species.

2. The determination method according to claim 1, wherein the one or more sRNA species exhibit a specific expression profile in the organism having a cell wall.

3. The determination method according to claim 1 or 2, wherein determining a presence state of an organism having a cell wall comprises determining a gross presence state of two or more organisms having a cell wall.

4. The determination method according to any one of claims 1 to 3, wherein detecting a presence state of the sRNA species comprises detecting an abundance of the sRNA species.

5. The determination method according to any one of claims 1 to 4, wherein the organism having a cell wall includes at least one selected from the group consisting of *Escherichia coli, Citrobacter freundii,* and *Salmonella gallinarum.*

6. The determination method according to any one of claims 1 to 4, wherein the organism having a cell wall includes a plant.

7. The determination method according to any one of claims 1 to 4, wherein the organism having a cell wall includes a verotoxin-producing bacterium.

8. The determination method according to any one of claims 1 to 7, wherein the measurement sample is obtained by collecting airborne matter, and determining a presence state of an organism having a cell wall comprises determining a presence state of an organism having a cell wall present in the air.

9. The determination method according to any one of claims 1 to 8, wherein immersing the measurement sample is performed at a temperature in a temperature range of from 0°C to 50°C.

10. The determination method according to any one of claims 1 to 9, wherein each of the one or more sRNA species has a length in a range of from 5 to 500 bases.

11. The determination method according to any one of claims 1 to 10, wherein the one or more sRNA species comprise at least one selected from the group consisting of EC-5p-36 having the nucleotide sequence of SEQ ID NO: 1, EC-3p-40 having the nucleotide sequence of SEQ ID NO: 2, EC-5p-79 having the nucleotide sequence of SEQ ID NO: 11, EC-3p-393 having the nucleotide sequence of SEQ ID NO: 12, fox_milRNA_5 having the nucleotide sequence of SEQ ID NO: 10, miR156 having the nucleotide sequence of SEQ ID NO: 4, and miR716b having the nucleotide sequence of SEQ ID NO: 5.

12. The determination method according to any one of claims 1 to 11, wherein the water solvent contains a nucleic acid amplifying reagent.

13. The determination method according to any one of claims 1 to 12, wherein detecting a presence state of the one or more sRNA species is performed using isothermal gene amplification.

14. The determination method according to claim 13, wherein the isothermal gene amplification is performed at a temperature in a temperature range of from 10°C to 40°C.

15. The determination method according to any one of claims 1 to 12, wherein detecting a presence state of the one or more sRNA species is performed using a PCR.

16. A method of identifying an organism having a cell wall, the method comprising:
preparing a liquid sample by immersing a measurement sample in a water solvent, or preparing a measurement sample that is a water solvent as a liquid sample; and
detecting a presence state of one or more sRNA species in the liquid sample,
wherein an organism having a cell wall present in the measurement sample is identified based on the presence state of the one or more sRNA species.

17. The identification method according to claim 16, wherein the one or more sRNA species exhibit a specific expression profile in accordance with an organism having a cell wall.

18. The identification method according to claim 16 or 17, wherein identifying an organism having a cell wall present in the measurement sample comprises identifying that at least one of two or more candidate organisms is present.

19. The identification method according to any one of claims 16 to 18, wherein detecting a presence state of the sRNA species comprises detecting an abundance of the sRNA species.

20. The identification method according to any one of claims 16 to 19, wherein the measurement sample is obtained by collecting airborne matter, and identifying an organism having a cell wall comprises identifying an organism having a cell wall present in the air.

21. The identification method according to any one of claims 16 to 20, wherein immersing the measurement sample is performed at a temperature in a temperature range of from 0°C to 50°C.

22. The identification method according to any one of claims 16 to 21, wherein each of the one or more sRNA species has a length in a range of from 5 to 500 bases.

23. The identification method according to any one of claims 16 to 22, wherein the one or more sRNA species comprise at least one selected from the group consisting of EC-5p-36 having the nucleotide sequence of SEQ ID NO: 1, EC-3p-40 having the nucleotide sequence of SEQ ID NO: 2, EC-5p-79 having the nucleotide sequence of SEQ ID NO: 11, EC-3p-393 having the nucleotide sequence of SEQ ID NO: 12, fox_milRNA_5 having the nucleotide sequence of SEQ ID NO: 10, miR156 having the nucleotide sequence of SEQ ID NO: 4, and miR716b having the nucleotide sequence of SEQ ID NO: 5.

24. The identification method according to any one of claims 16 to 23, wherein the water solvent contains a nucleic acid amplifying reagent.

25. The identification method according to any one of claims 16 to 24, wherein detecting a presence state of the one or more sRNA species is performed using isothermal gene amplification.

26. The identification method according to claim 25, wherein the isothermal gene amplification is performed at a temperature in a temperature range of from 10°C to 40°C.

27. The identification method according to any one of claims 16 to 24, wherein detecting a presence state of the one or more sRNA species is performed using a PCR.
